(19) Europäisches Patentamt
European Patent Office
Office européen des brevets

(11) EP 0 853 947 A1

# (12) EUROPEAN PATENT APPLICATION

(43) Date of publication:
**22.07.1998 Bulletin 1998/30**

(51) Int. Cl.[6]: **A61K 47/48**, A61L 33/00

(21) Application number: **97203538.0**

(22) Date of filing: **13.11.1997**

(84) Designated Contracting States:
**AT BE CH DE DK ES FI FR GB GR IE IT LI LU MC NL PT SE**
Designated Extension States:
**AL LT LV MK RO SI**

(30) Priority: **14.11.1996 NL 1004538**

(71) Applicant: **CORDIS EUROPA N.V.**
**NL-9301 LJ Roden (NL)**

(72) Inventor:
**Bos, Johannes Jacobus Gerardus**
**9956 PJ Den Andel (NL)**

(74) Representative:
**Van Someren, Petronella F. H. M. et al**
**Arnold & Siedsma,**
**Advocaten en Octrooigemachtigden,**
**Sweelinckplein 1**
**2517 GK Den Haag (NL)**

## (54) Matrix material comprising several bio-active materials

(57) The invention relates to a matrix material provided with a number of bio-active materials, in particular drugs, comprising a substrate, polyethyleneimine (PEI) coupled to the substrate, and several bio-active materials adhering to the polyethyleneimine and/or substrate.

The matrix material can be obtained by the following steps:

(a) preparing, if necessary, the substrate for the introduction of coupling groups;
(b) effecting contact between the substrate and a solution of PEI, if required in the presence of a carbodiimide; and
(c) subsequently effecting contact between the substrate treated with PEI and one or more solutions of bio-active materials, if required in the presence of a

EP 0 853 947 A1

## Description

This invention relates to a matrix material provided with a number of bio-active materials. The invention furthermore relates to a medical device coated with the matrix material and a method for manufacturing the material.

For many years now matrix materials have been used in medicine, for instance as coating for medical devices, in which fully or partly immobilized drugs have been contained. The purpose of these drugs is to locally reduce the biological responses developed by the body as a reaction to the presence of the medical device. The best known example in this respect is the catheter coated with heparin, whereby the heparin has to prevent the coagulation of blood on the catheter. Also medical devices which are to be implanted, in particular stents, appear to benefit from an immobilized heparin-coating.

In addition to heparin there are obviously also other drugs which have their own specific action and properties. The object of this invention is to combine the action and properties of several drugs in one matrix material.

This is achieved with the invention by providing a matrix material with a number of bio-active materials, in particular drugs, comprising a substrate, polyethyleneimine (PEI) coupled to the substrate and several bio-active materials adhering to the polyethyleneimine.

The matrix material according to the invention comprises at least two bio-active materials, but may obviously also comprise a 'cocktail' of drugs. Preferably the action of the bio-active materials is to suppress the immune response following implantation, transplantation et cetera, to prevent the occurrence of coagulation symptoms, or conversely, to stimulate clotting; to stimulate or inhibit cellular growth et cetera.

In one of the embodiments heparin and aprotinin are combined in the matrix material. The heparin will block both the intrinsic and extrinsic pathways of coagulation at the point where fibrinogen is converted to fibrin threads. This is achieved by inhibiting thrombin molecules by means of antithrombin III (ATIII). Aprotinin inhibits kallikrein, which is involved in the contact activation of the intrinsic pathway of coagulation. Aprotinin itself cannot prevent the clotting of blood and heparin itself is not capable of reducing the by-products that are generated by the intrinsic pathway of coagulation. However, the two of them combined prevent both clotting and a massive production of by-products generated by the intrinsic pathway, which production is triggered by contact with a foreign body like a medical device.

The different types of bio-active materials in the matrix material according to the invention may be immobilized to varying degrees. It is for instance possible that one of the drugs is permanently immobilized in the matrix, whereas the other drug is released during a longer or shorter period of time. In this way any combination of degrees of immobilization is conceivable. A matrix material according to the invention may for instance obtain a very dynamic action because the action of a drug which is released decreases, whereas a fully immobilized drug retains its action for a longer period of time. In this manner the action of the matrix material can be regulated according to requirements.

According to the invention this may even result in a reduction in the quantity of medication required to tranquillize negative biological responses induced by the introduction of a medical device. It consequently helps to improve the quality of life during the period of biological rejection, or of adaptation of the device introduced, and can increase the chance of acceptance of the device by the body.

The medical devices concerned may be introduced both for long-term procedures and short-term procedures, such as for instance implantation or diagnostic purposes respectively.

This invention furthermore provides a method for manufacturing a matrix material according to the invention. The method comprises the following steps:

(a) to prepare, if necessary, the substrate for the introduction of coupling groups;
(b) to effect contact between the substrate and a solution of PEI, if required in the presence of a carbodiimide; and
(c) to subsequently effect contact between the substrate treated with PEI and one or more solutions of bio-active materials, if required in the presence of a carbodiimide.

The preparation of the substrate entails in general the introduction of hydroxyl, oxygen and/or carboxylic acid groups. These groups are required in order to be able to couple the polyethyleneimine to the substrate. Several techniques, known as such already, exist in order to introduce such hydroxyl, oxygen and/or carboxylic acid groups. For instance through chemical/physical treatment by means of oxygen containing gas discharge, or through chemical treatment by means of liquid or gas-phase reactions, such as exposing the substrate to strong acidic or basic solutions or heat treatment in an oxygen-rich environment, through surface polymerization or a combination of those techniques.

In the case of gas discharge in an oxygen-containing environment various oxygen or oxygen-containing radicals are formed by ionization, recombination of an electron with an ion or by internal excitation, for instance due to collision with another molecule or interception of a photon. The chance that ions or exited neutrals will enter into a reaction with the environment (in this case for instance the surface of a catheter) is much greater than that the neutral forms in the ground state will do so. The radicals may react in different ways with the surface of the substrate provided, like for instance in the manner shown in the chemical equation below:

$$...\text{-}CH_2\text{-}... + O_2^* \rightarrow ...\text{-}CHOH\text{-}... + O$$

In this case "...-$CH_2$-..." represents part of the surface of the substrate. "$O_2^*$" is oxygen in exited state and "...-CHOH-..." is the substrate on which a hydroxyl group has formed. In a gas discharge any thinkable reaction may take place. The equilibria of these reactions determine the final outcome. This technique is described in greater detail in P.V. Narayanan, "Surface functionalization by RF plasma treatment of polymers for immobilization of bio-active molecules, J. Biomater. Sci Polymer Edn. 6(2), 181-193 (1994).

An example of a liquid-phase reaction is the so-called wet-chemical surface modification method of Pharmacia (O. Larm, R. Larsson & P. Olsson, "A new non-thrombogenic surface prepared by selective covalent binding of heparin via a reducing terminal residue", Biomater. Med. Devices Artif. Organs 11, 161-173 (1983). This method is for example suitable to bind heparin to intraocular lenses (IOL), made of Poly(methyl methacrylate) (PMMA, Perspex).

By effecting contact between the substrate and a monomer, and by activating the monomer to polymerization, a thin layer will be formed on the substrate. This method is called surface polymerization. The properties of the layer (both chemical and physical) may truly differ form those of the substrate. A plastic layer may for instance be arranged on metal. Activating monomers may be effected by external radiation with photons (usually UV-light), heating or adding a reaction component (for instance $H_2O$ with diisocyanates in order to form a polyurethane).

A well known example of surface polymerization is arranging PTFE or Teflon. The double bond of the monomer tetrafluoroethene is disrupted in a gas discharge, as a result of which a tetrafluoroethene radical is formed which will couple to a second tetrafluoroethene radical. In this way long chains are formed, which precipitate on all surfaces present, including that of the substrate exposed. This manner of surface treatment is described in P.V. Narayanan, "Frictional Characteristics of Radio Frequency Plasma Polymerized Films From Tetrafluoroethylene Monomer", Procs. ACS Division of Polymeric Materials: Science and Engineering 62, 553-557 (1990).

Surface polymerization is preferably carried out prior to the gas discharge or liquid or gas-phase reactions, and results in arranging a polymer layer on the original substrate. There are also several other methods of surface polymerization which can be used, such as immersing the substrate in a polymer-containing solution, arranging polymer layers or coverings around the substrate, or even by means of plasma-enhanced chemical vapour deposition (PECVD), in which case monomers are activated and are polymerized on the surface of the substrate.

Immersion of the substrate in a solution of molecules, of which at least a number already possess the required functional properties, is also a suitable method to create an appropriate surface.

In a particular embodiment of the invention a hydrogel has been arranged on the substrate. Hydrogels are often used as lubricants on medical devices, such as in coaxial systems, and may also contain a bio-active material. A well known example is the heparin-containing coating for the release of medication.

According to the invention a matrix material is consequently provided for the release of a bio-active material, in particular a drug, comprising a hydrogel arranged on a substrate, polyethyleneimine (PEI) coupled to at least a number of the hydrogel molecules, and more than one bio-active material adhering to the polyethyleneimine. The polyethyleneimine reacts on the one hand with the hydrogel, preferably with the carboxylic acid groups of the latter, and binds on the other hand the bio-active material.

The binding of PEI to the surface can for instance be effected via the diimide-chemistry, preferably on the basis of the carbodiimide ethyldimethylaminopropyl carbodiimide (EDC), but may also be based on ion forces or Van der Waals forces. The diimide-chemistry is as such well known and described for instance by Frederick Kurzer and K. Douraghi-Zadeh: "Advances in the Chemistry of Carbodiimides"; 67(2) 107-152 (1967); K.L. Carraway and D.E. Koshland, Jr.: "Carbodiimide Modification of Proteins"; pages 616-623; and M.A. Fox & J.K. Whitesell "Organic Chemistry", page 630, Jones and Bartlett Publishers International, 1994.

The coupling of the bio-active materials can also take place via diimide-chemistry. But also simply effecting contact between the matrix material treated with PEI and a solution of the bio-active material can be sufficient to bring about adhesion. The binding or coupling method employed depends on the required degree of immobilization.

When both the coupling of the PEI and the bio-active material takes place according to the principles of diimide-chemistry it can be schematically illustrated as follows:

O H H O EDC

$R_1$-C + N-....-N + C-$R_2$ ---->

OH H H HO

substrate (PEI) drug (for instance heparin)

$1^{st}$ STEP $2^{nd}$ STEP

O O

$R_1$ - C - N - (PEI) - N - C - $R_2$ + urea

H H (is removed)

Each bio-active material can be arranged separately in the matrix material, or combinations may be used.

This invention will be explained in greater detail in the following example, which is only provided by way of illustration and not with the intention of limiting the invention in any manner whatsoever.

**EXAMPLE**

Pieces of nylon catheters were heparinized according to the diimide-coupling scheme following functionalization by gas discharge and subsequent immobilization of polyethyleneimine. Because of the gas discharge hydroxyl, oxygen and carboxylic acid groups were created. To these the amino groups of the PEI were coupled. Next heparin was coupled, via its carboxylic acid groups, to the remaining amino groups of the PEI. In this manner a PEI-heparin matrix was formed. Subsequently the catheters were immersed in an aprotinin solution, which resulted in a PEI-heparin/aprotinin matrix material.

Catheters with a coating of only heparin and of heparin and aprotinin were placed for a certain period of time in a solution of thrombin and kallikrein respectively. Both these compounds are inactivated to a greater or lesser degree by the matrix material. After removal of the catheters a substrate was added which can be converted by thrombin and kallikrein. This is accompanied by discoloration of the substrate. The degree of this discoloration can be measured by establishing the absorption (or optic density), at for instance 405 nm. When much inhibition of thrombin or kallikrein has taken place, little is left in the solution to enable conversion of the substrate. This results in a low degree of absorption. The lower the absorption value, the higher the inactivation-action of the matrix material.

Figure 1 illustrates the inhibition of thrombin by a heparin and a heparin/aprotinin matrix respectively. Plotted on the x-axis is the incubation period. On the y-axis the optical density at 405 nm of the solution after removal of the catheters has been plotted. Figure 2 shows the same for kallikrein.

In vitro it appeared that the thrombin binding was slightly increased compared to a catheter only coated with heparin. The kallikrein binding increased however dramatically due to the presence of aprotinin compared to the samples which were only coated with heparin. Kallikrein is a protein which can activate factor VII, which in its turn activates factor VI and thus initiates the cascade of reactions which is known as the intrinsic pathway of coagulation of blood. This takes place in damaged tissue, or in cases of contact activation when blood enters into an interaction with foreign materials, such as the plastic substrate of an angiography catheter et cetera.

A heparin/aprotinin coating consequently effects the coagulation cascades at at least two points, that is to say at the beginning of the intrinsic pathway of coagulation by means of kallikrein binding and at the end of both the intrinsic and extrinsic pathway when thrombin inactivation in the presence of heparin and antithrombin III prevents the actual coagulation.

## Claims

1. Matrix material provided with a number of bio-active materials, in particular drugs, comprising a substrate, polyethyleneimine (PEI) coupled to the substrate, and several bio-active materials adhering to the polyethyleneimine and/or substrate.

2. Matrix material as claimed in claim 1, obtained by the following steps:

   (a) preparing, if necessary, the substrate for the introduction of coupling groups;
   (b) effecting contact between the substrate and a solution of PEI, if required in the presence of a carbodiimide; and
   (c) subsequently effecting contact between the substrate treated with PEI and one or more solutions of bio-active materials, if required in the presence of a carbodiimide.

3. Matrix material as claimed in claim 2, characterised in that the carbodiimide is ethyldimethylaminopropyl carbodiimide (EDC).

4. Matrix material as claimed in claim 2 or 3, characterised in that the preparation of the substrate involves the introduction of hydroxyl, oxygen and/or carboxylic acid coupling groups.

5. Matrix material as claimed in claim 4, characterised in that hydroxyl, oxygen and/or carboxylic acid groups are introduced through chemical/physical treatment by means of oxygen containing gas discharge, through chemical treatment by means of liquid or gas-phase reactions, such as exposing the substrate to strong acidic or basic solutions or heat treatment in an oxygen-rich environment, through surface polymerization or combinations of those techniques.

6. Matrix material as claimed in one of the claims 1-5, characterised in that the bio-active materials are heparin and aprotinin.

7. Medical device coated with a matrix material as claimed in claims 1-6.

8. Medical device as claimed in claim 7, characterised in that the medical device is a device to be implanted permanently or temporarily and is chosen from stents, transplants, vena cava filters, bone transplants, heart valves, artificial bones, artificial tissues, local obstructions.

9. Medical device as claimed in claim 7, characterised in that the medical device is intended for temporary contact with biological systems or parts thereof, such as blood or tissues, and is chosen from diagnostic catheters, intervention catheters, such as dilatation catheters, microcatheters, guide wires, catheter introduction systems, such as the so-called catheter sheath introducers (CSI), temporary vena cava filters, bio-reabsorbable stents, implants, bones.

10. Medical device as claimed in claim 7, characterised in that the medical device is intended for ex vivo use, such as heart pumps or other external blood-circulation devices, wherein the matrix material has been arranged on the tubing of the device.

11. Use of the matrix material as claimed in one of the claims 1-6 for at least partly coating a medical device as claimed in one of the claims 7-10.

12. Method for manufacturing a matrix material as claimed in one of the claims 1-6 comprising:

   (a) preparing, if necessary, the substrate for the introduction of coupling groups;
   (b) effecting contact between the substrate and a solution of PEI, if required in the presence of a carbodiimide; and
   (c) subsequently effecting contact between the substrate treated with PEI and one or more solutions of bio-active materials, if required in the presence of a carbodiimide.

13. Method as claimed in claim 12, characterised in that the carbodiimide is ethyldimethylaminopropyl carbodiimide (EDC).

**14.** Method as claimed in claim 12 or 13, characterised in that the preparation of the substrate involves the introduction of hydroxyl, oxygen and/or carboxylic acid coupling groups.

**15.** Method as claimed in claim 14, characterised in that hydroxyl, oxygen and/or carboxylic acid groups are introduced through chemical/physical treatment by means of oxygen containing gas discharge, through chemical treatment by means of liquid or gas-phase reactions, such as exposing the substrate to strong acidic or basic solutions or heat treatment in an oxygen-rich environment, through surface polymerization or a combination of those techniques.

**16.** Methods as claimed in one of the claims 12-15, characterised in that the bio-active materials are heparin and aprotinin.

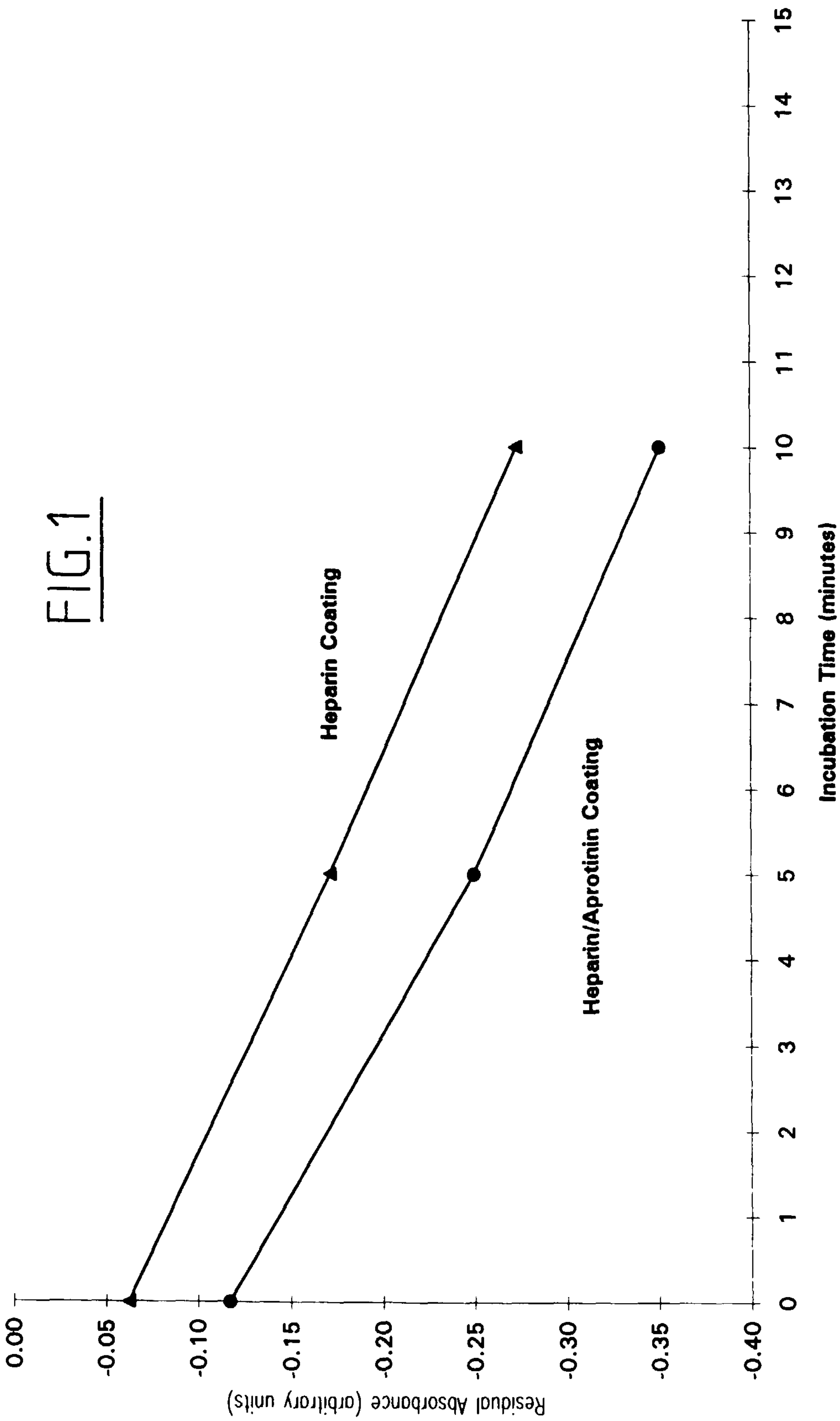
Heparin Coating
Heparin/Aprotinin Coating
Incubation Time (minutes)
Residual Absorbance (arbitrary units)
0.00
-0.05
-0.10
-0.15
-0.20
-0.25
-0.30
-0.35
-0.40
0
1
2
3
4
5
6
7
8
9
10
11
12
13
14
15

FIG.1

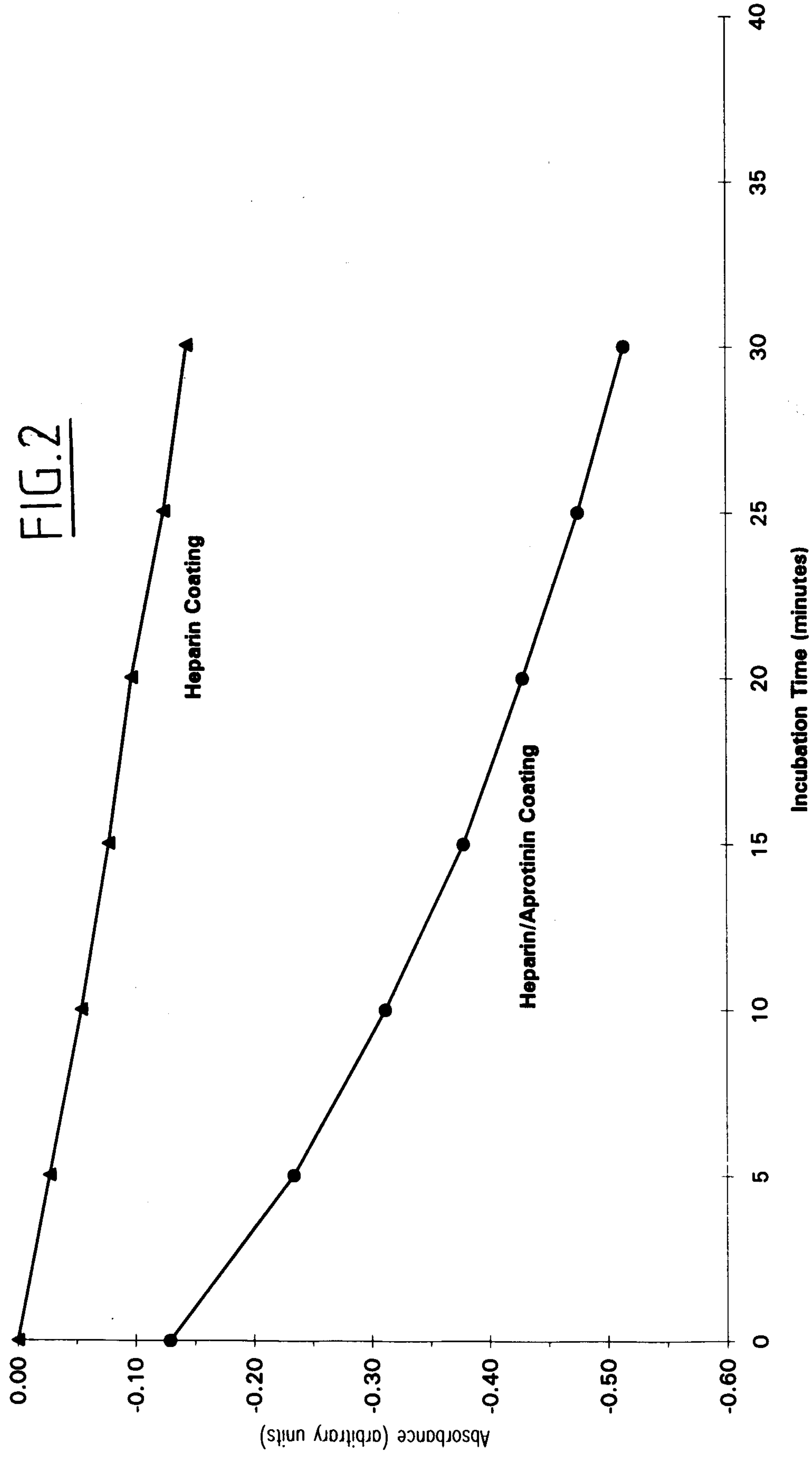
Heparin Coating
Heparin/Aprotinin Coating
Incubation Time (minutes)
Absorbance (arbitrary units)
0.00
-0.10
-0.20
-0.30
-0.40
-0.50
-0.60
0
5
10
15
20
25
30
35
40

FIG.2

European Patent Office

# EUROPEAN SEARCH REPORT

Application Number
EP 97 20 3538

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 125, no. 14, 30 September 1996 Columbus, Ohio, US; abstract no. 177320, NJIRI, CHSATO ET AL: "Improved non-thrombogenicity of heparin immobilized and sulfonated polyurethane: in vitro evaluation using epifluorescent video microscopy" XP002035331 * abstract * & ADV. BIOMATER. BIOMED. ENG. DRUG DELIVERY SYST., 'IKETANI CONF. BIOMED. POLYM.!, 5TH (1996), MEETING DATE 1995, 247-248. EDITOR(S): OGATA, NAOYA. PUBLISHER: SPRINGER, TOKYO, JAPAN. CODEN: 63CXA6, 1996, --- | 1-16 | A61K47/48<br>A61L33/00 |
| X | GB 2 257 147 A (CORDIS CORP) 6 January 1993 * page 2, line 6 - line 24 * * page 5, line 8 - line 37 * * page 9, line 24 - line 34; claims 1,8 * --- | 1-15 | **TECHNICAL FIELDS SEARCHED (Int.Cl.6)**<br>A61K<br>A61L |
| X | US 5 132 108 A (NARAYANAN PALLASSANA V ET AL) 21 July 1992 * column 4, line 25 - line 54; claims 1,8,11,13 * --- | 1-16 | |
| X | O. LARM ET AL.: "A NEW NON-THROMBOGENIC SURFACE PREPARED BY SELECTIVE COVALENT BINDING OF HEPARIN VIA A MODIFIED REDUCING TERMINAL RESIDUE." BIOMAT., MED. DEV. , ART. ORG., vol. 11, no. 2&3, 1983, pages 161-173, XP002035330 * page 163 * --- -/-- | 1,2, 6-12,16 | |

The present search report has been drawn up for all claims

| Place of search | Date of completion of the search | Examiner |
|---|---|---|
| THE HAGUE | 19 May 1998 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 97 20 3538

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 351 314 A (TERUMO CORP) 17 January 1990<br>* page 3, line 48 - line 58 *<br>* page 4, line 53 - line 63 *<br>* page 5, line 8 - line 28 *<br>--- | 1,7-11 | |
| X | US 4 786 556 A (HU CAN B ET AL) 22 November 1988<br>* column 2, line 55 - column 3, line 41; claims 1-4,11 *<br>--- | 1,2, 5-12,15, 16 | |
| X | WO 83 02954 A (VENTREX LAB INC) 1 September 1983<br>* page 20, line 8 - line 20 *<br>* page 2, paragraph 2 *<br>* page 9, paragraph 2 *<br>--- | 1-3,7, 11-13 | |
| X | US 5 532 311 A (SIRVIO LARRY M ET AL) 2 July 1996<br>* column 2, line 5 - line 14 *<br>* column 2, line 33 - line 44 *<br>* column 3, line 19 - line 23 *<br>--- | 1,2, 4-12, 14-16 | TECHNICAL FIELDS SEARCHED (Int.Cl.6) |
| P,X | WO 97 07834 A (MEDTRONIC INC) 6 March 1997<br>* page 3, line 25 - page 4, line 16; claims *<br>----- | 1-16 | |

The present search report has been drawn up for all claims

| Place of search | Date of completion of the search | Examiner |
|---|---|---|
| THE HAGUE | 19 May 1998 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)